# EUROPEAN PATENT APPLICATION

(11) **EP 4 417 055 A1**
(43) Date of publication of application: **21.08.2024**
(21) Application number: 21960531.8
(22) Date of filing: 11.10.2021
(51) Int. Cl.: A01N 59/00, A01H 3/00, A01P 21/00, B01J 19/08

(54) **METHOD FOR INDUCING PLANT DISEASE RESISTANCE, DEVICE FOR INDUCING PLANT DISEASE RESISTANCE, AND AGENT FOR INDUCING PLANT DISEASE RESISTANCE**

(71) Applicant: Tohoku University, Sendai-shi, Miyagi 980-8577 (JP)
(72) Inventor: KANEKO, Toshiro, Sendai-shi, Miyagi 980-8577 (JP); TAKASHIMA, Keisuke, Sendai-shi, Miyagi 980-8577 (JP); SASAKI, Shota, Sendai-shi, Miyagi 980-8577 (JP); TAKAHASHI, Hideki, Sendai-shi, Miyagi 980-8577 (JP); ANDO, Sugihiro, Sendai-shi, Miyagi 980-8577 (JP); HIGASHITANI, Atsushi, Sendai-shi, Miyagi 980-8577 (JP); DAIGAKU, Yasukazu, Sendai-shi, Miyagi 980-8577 (JP)
(74) Representative: Plougmann Vingtoft a/s
(86) International application number: PCT/JP2021/037518
(87) International publication number: WO 2023/062667

(57) **Abstract**

[Problem] The present invention provides a plant disease resistance inducing method and a plant disease resistance inducing device, as well as a plant disease resistance inducing agent, which can induce a systemic resistance without inhibiting the plant growth in a relatively easy and inexpensive manner and with little environmental loads.

[Solution] A plant is exposed to a gas containing dinitrogen pentoxide to induce the disease resistance. It is particularly preferable that dinitrogen pentoxide is produced using a plasma generated with air as a source gas, and a plant is exposed to a gas containing dinitrogen pentoxide.

## Description

### Field of the Invention

The present invention relates to a plant disease resistance inducing method and a plant disease resistance inducing device, as well as a plant disease resistance inducing agent.

### Description of Related Art

Plants have defense functions against attacks by pathogens such as filamentous fungi, bacteria, and viruses, and against impairments such as insect damages due to harmful insects. Among these defense functions, two types of defense responses: systemic acquired resistance (SAR); and induced systemic resistance (ISR), are known, which induce a resistance in a whole plant body (e.g., see Patent Literatures 1 and 2).

SAR mainly induces a resistance against pathogens, and salicylic acid (SA) is involved in signaling of SAR. On the other hand, ISR mainly induces a resistance against impairments such as insect damages, and jasmonic acid (JA) and ethylene (ET) are involved in signaling of ISR. Conventionally, there have been proposed methods using various compounds to induce resistances by SAR or ISR (e.g., see Patent Literatures 1 and 2). Also, there have been proposed methods to induce SAR defense response using ozone (e.g., see Non-Patent Literature 1).

It is known that, in the ISR defense response, Ca²⁺ signals (injury information) are transmitted from cells and tissues of leaves injured due to insect damages or the like, through sieve tubes, and to the whole body, so that the systemic defense mechanism is activated even in distant healthy organs by synthesizing jasmonic acid that is one of plant hormones (e.g., see Non-Patent Literature 2). As metabolic pathways related to systemic defense mechanisms, antimicrobial peptide production and tryptophan metabolic pathways activated by jasmonic acid (JA)/ethylene (ET) are known. Examples of known genes related to each of these pathways include PDF1.2, ORA59, PAD3, CYP71A12, NIT2, and WRKY26 (e.g., see Non-Patent Literature 3).

The inventors of the present invention have developed an injector and a sterilization/extermination method which can sterilize and exterminate pathogens and harmful insects adhering to plants, soil, fertilizers, or the like without using pesticides by producing reactive species such as OH radicals and peroxynitrite (HOONO) using plasma and injecting a liquid containing the produced reactive species (e.g., see Patent Literatures 3 to 6). These devices and methods are intended not to induce plant resistance but to directly sterilize and exterminate pathogens and harmful insects.

### Citation List

### Patent Literatures

Patent Literature 1: WO 2016/006351
Patent Literature 2: JP-A-2017-61460
Patent Literature 3: JP 5909831
Patent Literature 4: WO 2018/043468
Patent Literature 5: JP-A-2019-40863
Patent Literature 6: JP-A-2020-130085

### Non-Patent Literatures

Non-Patent Literature 1: YOGESH K. SHARMA et al., "Ozone-induced responses in Arabidopsis thaliana: The role of salicylic acid in the accumulation of defense-related transcripts and induced resistance", Proceedings of the National Academy of Sciences of the United States of America, May 1996, Vol. 93, p. 5099-5104
Non-Patent Literature 2: Masatsugu Toyota et al., "Glutamate triggers long-distance, calcium-based plant defense signaling", Science, 2018, 361, p. 1112-1115
Non-Patent Literature 3: Frerigmann et al., "Regulation of pathogen-triggered tryptophan metabolism in Arabidopsis thaliana by MYB transcription factors and indole glucosinolate conversion products", Molecular Plant, 2016, 9, p. 682-695

### Summary of the Invention

The methods for inducing SAR and ISR defense reactions using various compounds, as described in Patent Literatures 1 and 2, have had a problem that costs and labors required for producing the compounds are increased. In addition, some of the compounds for use have had a problem that the compounds remain in the environment to increase environmental loads. The method for inducing the SAR defense response by ozone described in Non-Patent Literature 1 has had a problem that the response to ozone is so intense that it causes significant damage to the plant, for example, leaves exposed to ozone wither, resulting in a high risk of inhibiting the plant growth.

The present invention has been made with a focus on the above problem, and an object of the present invention is to provide a plant disease resistance inducing method and a plant disease resistance inducing device, as well as a plant disease resistance inducing agent, which can induce a systemic resistance without inhibiting the plant growth in a relatively easy and inexpensive manner and with little environmental loads.

In order to achieve the object described above, the plant disease resistance inducing method according to the present invention is characterized in that a plant is exposed to a gas containing dinitrogen pentoxide (N₂O₅) to induce the disease resistance.

The plant disease resistance inducing device according to the present invention is characterized in that it can expose a plant to a gas containing dinitrogen pentoxide to induce a disease resistance.

In the plant disease resistance inducing method according to the present invention, it is particularly preferable that dinitrogen pentoxide described above is produced using a plasma generated with air as a source gas, and a plant is exposed to a gas containing dinitrogen pentoxide. It is particularly preferable that the plant disease resistance inducing device according to the present invention includes a dinitrogen pentoxide production portion that can produce dinitrogen pentoxide using a plasma generated with air as a source gas, in which the plant can be exposed to the gas containing dinitrogen pentoxide produced in the dinitrogen pentoxide production portion.

The plant disease resistance inducing method according to the present invention is suitably implemented using the plant disease resistance inducing device according to the present invention. The plant disease resistance inducing method and device according to the present invention can induce a calcium ion response in plant cells by exposing a plant to dinitrogen pentoxide. Also, the method and device can induce expression of a resistance gene related to plant resistance induction by exposing a plant to dinitrogen pentoxide. This makes it possible to induce the systemic ISR defense response such as jasmonic acid synthesis through the expression of the resistance gene, even on sites far from sites exposed to dinitrogen pentoxide.

The plant disease resistance inducing method and device according to the present invention can be used for any plant and can induce the ISR defense response. Also, the plant disease resistance inducing method and device according to the present invention can induce the systemic disease resistance without using pesticides by using dinitrogen pentoxide. Because of use of dinitrogen pentoxide, leaves exposed to dinitrogen pentoxide do not wither unlike the case with ozone, and growth of the plant exposed to dinitrogen pentoxide is not inhibited. Since dinitrogen pentoxide that has not been absorbed by the plant promptly reacts with water and changes into nitrate ions with extremely low toxicity, a safe and clean plant growth environment can be maintained with little environmental loads and without pollution of atmosphere or soil.

In the plant disease resistance inducing method and device according to the present invention, it is preferable to expose a plant to a gas containing dinitrogen pentoxide under a condition optimal for growth of individual plant. In particular, since a high moisture content increases the possibility to impair the plant and a low moisture content decreases the effectiveness of inducing the plant resistance, it is preferable to expose the plant to the gas containing dinitrogen pentoxide at an appropriate moisture content for the plant species. A concentration of dinitrogen pentoxide in the gas for the exposure is preferably 0.1 ppm to 1000 ppm, and preferably about 200 ppm or lower, but should be selected as appropriate depending on the plant species. An exposure time is preferably 10 seconds to 1 hour, and particularly preferably within 5 minutes, but should be selected as appropriate depending on the plant species.

In the plant disease resistance inducing method and device according to the present invention, dinitrogen pentoxide may be produced by any method or device, but it is preferable to use dinitrogen pentoxide produced using a plasma generated with, as a source gas, a gas containing nitrogen and oxygen, such as air. In particular, dinitrogen pentoxide is produced using a plasma generated with air as a source gas by atmospheric discharge, so that the systemic resistance can be induced in a relatively easy and inexpensive manner. The plant disease resistance inducing method and device according to the present invention can be used as a plant calcium ion response inducing method and device respectively, or a plant resistance gene inducing method and device respectively.

For the plant disease resistance inducing method and device according to the present invention, preferable examples of resistance genes whose expression is induced by exposure to dinitrogen pentoxide include genes such as JAZ5, JAZ7, OPR3, PDF1.2, ORA59, PAD3, CYP71A12, NIT2, and WRKY26 related to synthesis of jasmonic acid, genes of transcription factors related to jasmonic acid signals, antimicrobial peptide genes, and genes related to biosynthesis of an antimicrobial substance camalexin. When the expression of these resistance genes is induced, the ISR defense response such as jasmonic acid synthesis can be induced.

The plant disease resistance inducing method according to the present invention includes: a NOₓ production step of producing nitrogen oxides by generating a plasma using, as a source gas, a gas containing nitrogen and oxygen; an ozone production step of producing ozone by generating a plasma using, as a source gas, a gas containing nitrogen and oxygen, or a gas obtained after the plasma generation in the NOₓ production step; a mixing step of producing dinitrogen pentoxide by preserving the nitrogen oxides produced in the NOₓ production step and ozone produced in the ozone production step in a same space for a predetermined time; and a dilution step of diluting dinitrogen pentoxide by adding a gas containing nitrogen and oxygen, conditioned such that its moisture content after dilution is lower than a predetermined moisture content, to dinitrogen pentoxide produced in the mixing step. Preferably, a plant is exposed to a gas containing dinitrogen pentoxide produced in the dilution step.

The plant disease resistance inducing device according to the present invention includes: a NOₓ production portion that can produce nitrogen oxides by generating a plasma using, as a source gas, a gas containing nitrogen and oxygen; an ozone production portion that can produce ozone by generating a plasma using, as a source gas, a gas containing nitrogen and oxygen, or a gas obtained after the plasma generation in the NOₓ production portion; a mixing portion that can produce dinitrogen pentoxide by preserving the nitrogen oxides produced in the NOₓ production portion and ozone produced in the ozone production portion in a same space for a predetermined time; and a dilution portion that dilutes dinitrogen pentoxide by adding a gas containing nitrogen and oxygen, conditioned such that its moisture content after dilution is lower than a predetermined moisture content, to dinitrogen pentoxide produced in the mixing portion. Preferably, the device is configured such that a plant can be exposed to the gas containing dinitrogen pentoxide produced in the dilution portion.

When using these nitrogen oxides and ozone, dinitrogen pentoxide can be produced by preserving the nitrogen oxides and ozone produced by the plasma in a same space for a predetermined time to react the nitrogen oxides and ozone. Preferably, dinitrogen pentoxide is produced at at a high concentration so as not to decompose the produced dinitrogen pentoxide into NO₂ or NO₃. At this time, since the exposure to a high concentration of dinitrogen pentoxide may impair the plant, a gas containing nitrogen and oxygen is added to the produced dinitrogen pentoxide to dilute dinitrogen pentoxide, so that the plant can be exposed to dinitrogen pentoxide at a concentration suitable for the plant to prevent impairment of the plant. Since dinitrogen pentoxide is highly sensitive to water, the produced dinitrogen pentoxide is diluted by adding, to dinitrogen pentoxide, a gas containing nitrogen and oxygen, conditioned such that its moisture content after dilution is lower than a predetermined moisture content, so that dinitrogen pentoxide can be prevented from reacting with water and from changing into nitric acid. Preferably, the predetermined moisture content described above is a moisture content that prevents progression of the reaction between dinitrogen pentoxide and water. Examples of the nitrogen oxides produced by the plasma include NO, NO₂, and N₂O.

In the case using these nitrogen oxides and ozone, when the gas containing nitrogen and oxygen as a source gas for producing ozone is used, the separately produced nitrogen oxides and ozone are put together into a same space and preserved for a predetermined time. When a gas obtained after production of the nitrogen oxides by a plasma is used as a source gas for producing ozone, the gas obtained after production of ozone contains the nitrogen oxides and ozone, and therefore the device is configured such that the gas obtained after production of ozone is put into one space and preserved for a predetermined time.

When using these nitrogen oxides and ozone, the plant disease resistance inducing method according to the present invention may be configured such that, in the mixing step, the gas containing the nitrogen oxides produced in the NOₓ production step and the gas containing ozone produced in the ozone production step are introduced into a tube from an opening on one end of the tube, and, after a predetermined time has elapsed, exhausted from an opening on the other end of the tube. The plant disease resistance inducing device according to the present invention may be configured such that the mixing portion includes a tube, and the gas containing the nitrogen oxides produced in the NOₓ production portion and the gas containing ozone produced in the ozone production portion can be introduced into the tube from an opening on one end of the tube, and, after a predetermined time has elapsed, exhausted from an opening on the other end of the tube. In this case, the nitrogen oxides and ozone are reacted in the tube, and dinitrogen pentoxide produced by the reaction can be exhausted from the opening on the other end of the tube. Preferably, the tube has a length and a diameter that allow a predetermined reaction time to be ensured so that the nitrogen oxides and ozone introduced from the opening on one end can react while passing through the inside of the tube.

The plant disease resistance inducing agent according to the present invention is characterized in that it includes a gas containing dinitrogen pentoxide.

The plant disease resistance inducing agent according to the present invention is suitably used by the plant disease resistance inducing method and device according to the present invention. The plant disease resistance inducing agent according to the present invention can be ejected onto a plant to induce a calcium ion response in plant cells. The agent can also induce a resistance gene expression of the plant. This makes it possible to induce the systemic ISR defense response such as jasmonic acid synthesis through the expression of the resistance gene, even on sites far from sites exposed to dinitrogen pentoxide.

Since the plant disease resistance inducing agent according to the present invention includes dinitrogen pentoxide, the agent can induce a systemic disease resistance without using pesticides. Furthermore, leaves exposed to dinitrogen pentoxide do not wither unlike the case with ozone, and growth of the plant exposed to dinitrogen pentoxide is not inhibited. Since the agent is manufactured using a plasma generated with air as a source gas, the systemic resistance can be induced in a relatively easy and inexpensive manner. The plant disease resistance inducing agent according to the present invention can be used as a plant calcium ion response inducing agent or a plant resistance gene inducing agent.

The present invention makes it possible to provide a plant disease resistance inducing method and a plant disease resistance inducing device, as well as a plant disease resistance inducing agent, which can induce systemic resistance without inhibiting the plant growth in a relatively easy and inexpensive manner and with little environmental loads.

### Brief Description of the Drawings

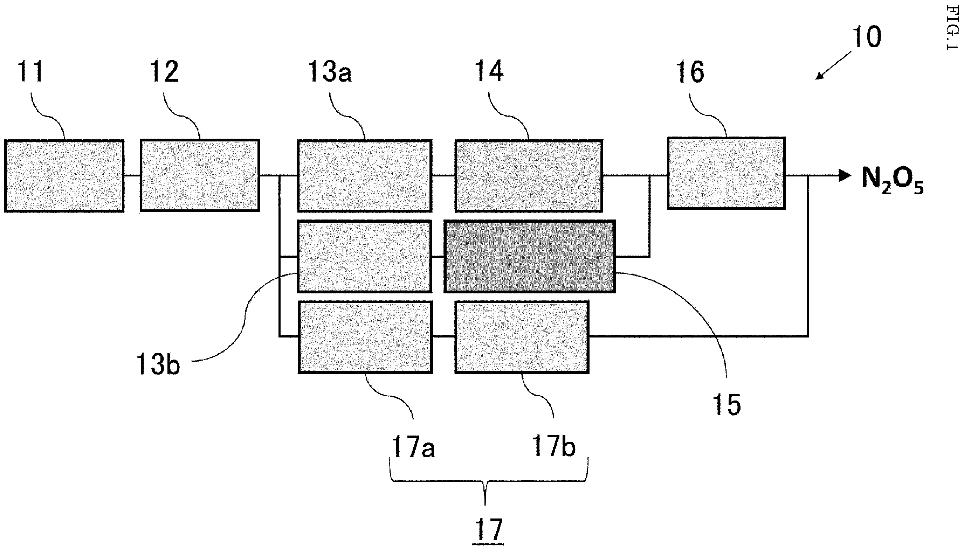

FIG. 1 is a block diagram illustrating a plant disease resistance inducing device according to the embodiment of the present invention.

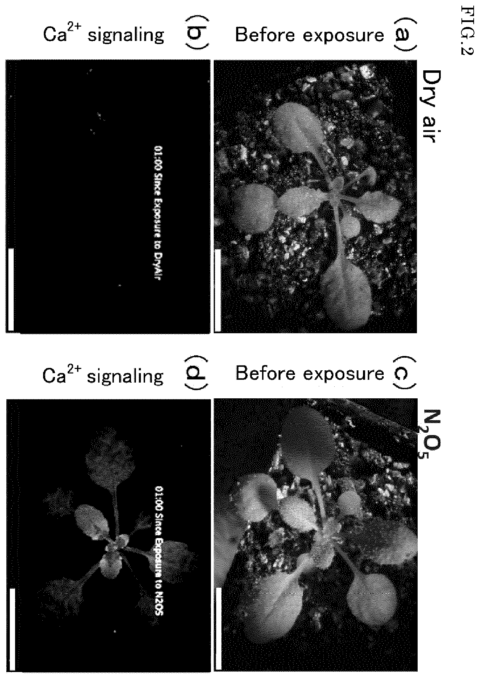

FIG. 2 includes photographs of observation results in a plant disease resistance inducing method according to the embodiment of the present invention, presenting (a) *Arabidopsis thaliana* before systemic dry air exposure, (b) Ca²⁺ signaling of *Arabidopsis thaliana* 60 seconds after the dry air exposure, (c) *Arabidopsis thaliana* before systemic dinitrogen pentoxide exposure, and (d) Ca²⁺ signaling of *Arabidopsis thaliana* 60 seconds after the dinitrogen pentoxide exposure.

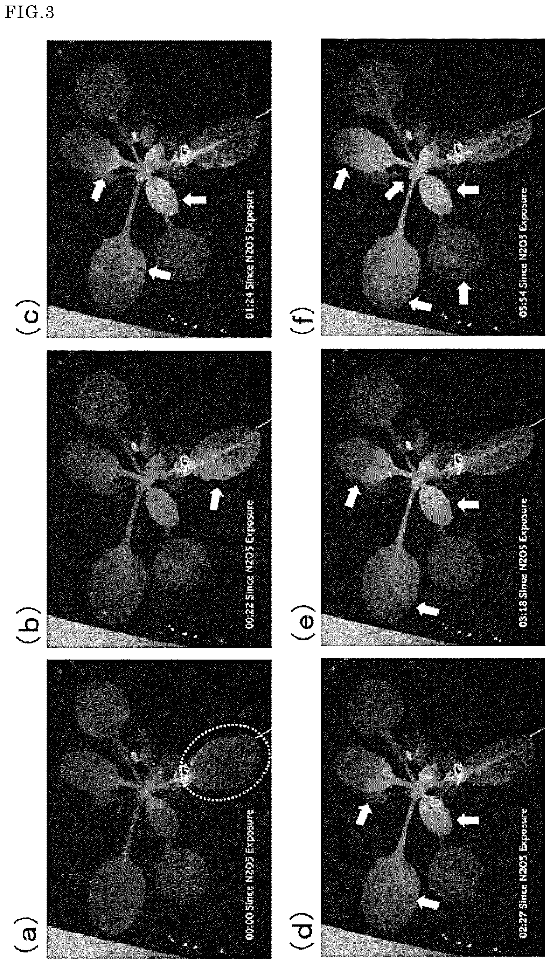

FIG. 3 includes photographs of observation results in a plant disease resistance inducing method according to the embodiment of the present invention, presenting Ca²⁺ signaling states of *Arabidopsis thaliana* observed by exposing one leaf (surrounded by dotted line in (a)) to dinitrogen pentoxide: (a) immediately after the exposure, (b) 22 seconds after the exposure, (c) 1 minute and 24 seconds after the exposure, (d) 2 minutes and 27 seconds after the exposure, (e) 3 minutes and 18 seconds after the exposure, and (f) 5 minutes and 54 seconds after the exposure.

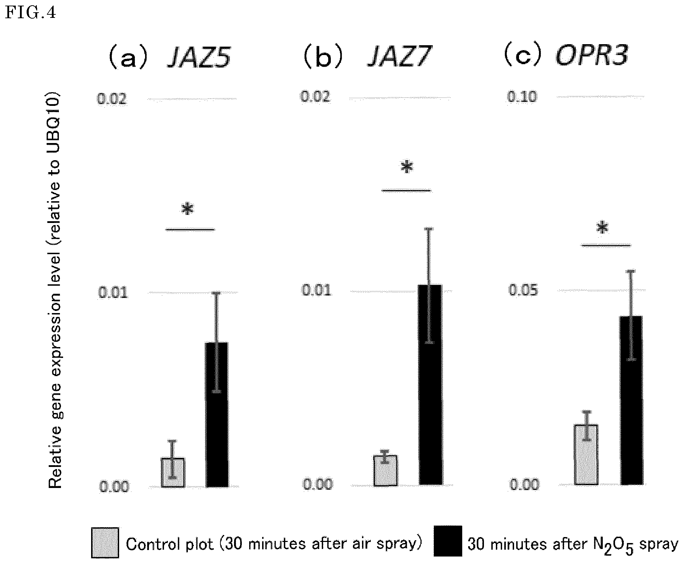

FIG. 4 includes graphs presenting expression levels of (a) JAZ5, (b) JAZ7, and (c) OPR3 of a leaf showing Ca²⁺ signaling, 30 minutes after exposure to dinitrogen pentoxide or dry air (control plot) in the plant disease resistance inducing method according to the embodiment of the present invention in FIG. 3.

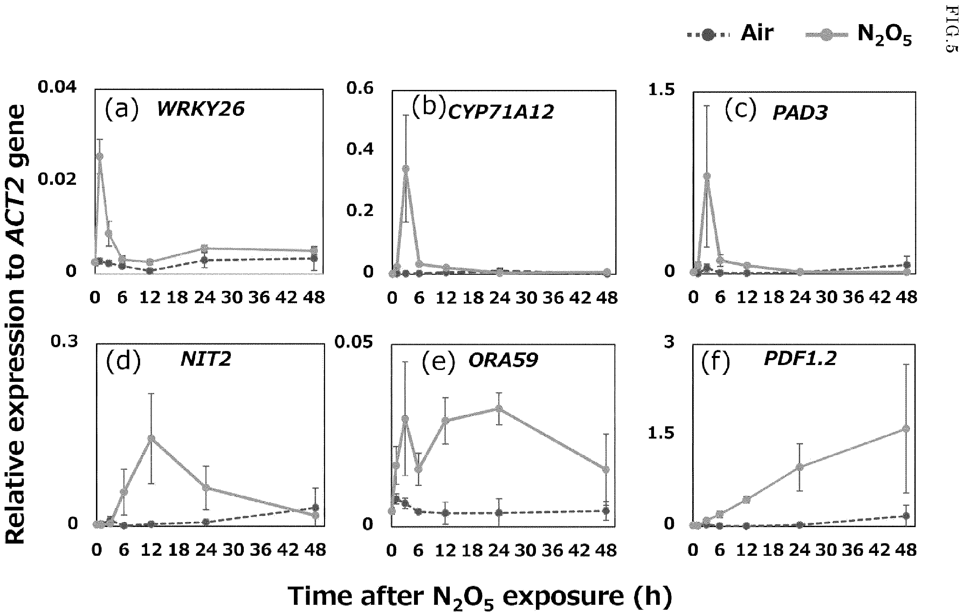

FIG. 5 includes graphs presenting time-dependent changes in expression levels (Relative expression to ACT2 gene) of (a) WRKY26, (b) CYP71A12, (c) PAD3, (d) NIT2, (e) ORA59, and (f) PDF1.2 observed by exposing *Arabidopsis thaliana* to dinitrogen pentoxide or dry air (comparative example) in the plant disease resistance inducing method according to the embodiment of the present invention.

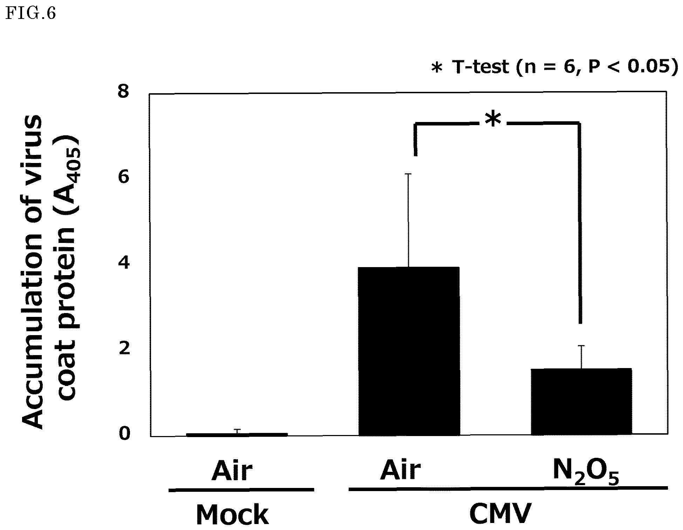

Fig. 6 is a graph presenting viral loads (Accumulation of virus coat protein) 2 days after a treatment in which *Arabidopsis thaliana* is exposed to dinitrogen pentoxide or dry air (comparative example) and then inoculated with a cucumber mosaic virus (CMV) macula lutea strain or no virus (Mock) in the plant disease resistance inducing method according to the embodiment of the present invention.

### Detailed Description of the Invention

The embodiment of the present invention will be explained below with reference to the drawings and examples.

FIG. 1 to FIG. 6 present the plant disease resistance inducing method and the plant disease resistance inducing device, as well as the plant disease resistance inducing agent according to the embodiment of the present invention.

As illustrated in FIG. 1, a plant disease resistance inducing device 10 includes a source gas introduction portion 11, a source gas production portion 12, two flow control portions 13a and 13b, a NOₓ production portion 14, an ozone production portion 15, a mixing portion 16, and a dilution portion 17.

The source gas introduction portion 11 is composed of a compressor and is configured to compress air as a source gas and feed the compressed air to the source gas production portion 12. The source gas production portion 12 is configured to dehydrate air fed from the source gas introduction portion 11 to adjust a moisture content. The source gas production portion 12 is configured to feed the moisture content-adjusted air to each of the flow control portions 13a and 13b and the dilution portion 17. In a specific example illustrated in FIG. 1, the source gas production portion 12 is configured to adjust the moisture content of the introduced air to 1×10¹⁵ cm⁻³ (about 40 ppm) or lower. Although the source gas is composed of air, it may be any gas other than air as long as it contains nitrogen and oxygen.

The flow control portion 13a is connected to the source gas production portion 12 and the NOₓ production portion 14, and the flow control portion 13b is connected to the source gas production portion 12 and the ozone production portion 15. Each of the flow control portions 13a and 13b is configured to receive air whose moisture content has been adjusted in the source gas production production 12, to adjust a flow volume and a flow rate of air, and to feed air to the NOₓ production portion 14 or the ozone production portion 15.

The NOₓ production portion 14 is configured to generate a plasma using, as a source gas, air fed from the flow control portion 13a to produce ozone. An electric power supplied at this time is adjusted to generate a plasma at 200°C or higher, so that nitrogen oxides such as NO, NO₂, and N₂O can be produced.

The ozone production portion 15 is configured to generate a plasma using, as a source gas, air fed from the flow control portion 13b to produce ozone. An electric power supplied at this time is adjusted to generate a plasma at 50°C or lower, so that ozone can be produced.

The mixing portion 16 has an elongated tube, and an opening on one end of the tube is connected to the NOₓ production portion 14 and the ozone production portion 15. The mixing section 16 is configured to receive, thereinside, the gas containing the nitrogen oxides produced in the NOₓ production portion 14 and the gas containing ozone produced in the ozone production portion15 and to exhaust the gases from an outlet on the other end. The tube has a length and an inner diameter that allow a reaction time to be ensured so that the introduced nitrogen oxides and ozone can react to produce dinitrogen pentoxide while passing through the inside of the tube. Thus, the mixing portion 16 is configured to exhaust dinitrogen pentoxide produced in the tube from the outlet.

In the specific example illustrated in FIG. 1, the tube has a length of 5 m to 50 m and an inner diameter of 4 mm to 10 mm so as to ensure a time of 10 seconds to 100 seconds for producing dinitrogen pentoxide. The tube is configured to allow the nitrogen oxides and ozone to pass through thereinside at 100°C or lower. The mixing portion 16 only needs to ensure a time required for producing dinitrogen pentoxide, and the time may be ensured by pressurizing or cooling down the gas containing nitrogen oxides and the gas containing ozone.

The dilution portion 17 is connected to the source gas production portion 12 and the outlet of the mixing portion 16, and is configured to dilute dinitrogen pentoxide produced in the mixing portion 16. The dilution portion 17 includes a dryer 17a and a flow controller 17b. The dryer 17a is configured to receive air whose moisture content has been adjusted in the source gas production portion 12 and further dry the air so that the moisture content after the dilution is less than a predetermined moisture content. Herein, the predetermined amount refers to a moisture content that prevents progression of the reaction between dinitrogen pentoxide and water. The flow controller 17b is configured to dilute dinitrogen pentoxide produced in the mixing portion 16 by adding air dried by the dryer 17a to dinitrogen pentoxide. The dilution portion 17 can dilute dinitrogen pentoxide to a desired concentration by adjusting the flow volume and flow rate of air.

The plant disease resistance inducing device 10 is configured such that a plant can be exposed to a gas containing dinitrogen pentoxide produced in the dilution portion 17. The gas containing dinitrogen pentoxide produced in this way constitutes the plant disease resistance induing agent according to the embodiment of the present invention. The region from the source gas introduction portion 11 to the dilution portion 17 constitutes the dinitrogen pentoxide production portion.

Next, the effects will be explained.

The plant disease resistance inducing method according to the embodiment of the present invention is suitably implemented by the plant disease resistance inducing device 10. The plant disease resistance inducing method and the plant disease resistance inducing device 10 according to the embodiment of the present invention can induce a calcium ion response in plant cells by exposing the plant to dinitrogen pentoxide. Also, the method and device can induce resistance gene expression related to plant resistance induction by exposing the plant to dinitrogen pentoxide. Examples of resistance genes whose expression is induced include genes such as JAZ5, JAZ7, OPR3, PDF1.2, ORA59, PAD3, CYP71A12, NIT2, and WRKY26 related to synthesis of jasmonic acid, genes of transcription factors related to jasmonic acid signals, antimicrobial peptide genes, and genes related to biosynthesis of an antimicrobial substance camalexin. These genes make it possible to induce the systemic ISR defense response such as jasmonic acid synthesis through the expression of the resistance gene, even on sites far from sites exposed to dinitrogen pentoxide.

The plant disease resistance inducing method and the plant disease resistance inducing device 10 according to the embodiment of the present invention can be used for any plant to induce the ISR defense response. Also, the plant disease resistance inducing method and the plant disease resistance inducing device 10 according to the embodiment of the present invention can induce a systemic disease resistance without using pesticides by utilizing dinitrogen pentoxide. Because of use of dinitrogen pentoxide, leaves exposed to dinitrogen pentoxide do not wither unlike the case with ozone, and growth of the plant exposed to dinitrogen pentoxide is not inhibited. Since dinitrogen pentoxide that has not been absorbed by the plant promptly reacts with water and changes into nitrate ions with extremely low toxicity, a safe and clean plant growth environment can be maintained with little environmental loads and without pollution of atmosphere or soil.

The plant disease resistance inducing method and the plant disease resistance inducing device 10 according to the embodiment of the present invention can produce dinitrogen pentoxide by preserving the nitrogen oxides and ozone produced by the plasma in a same space for a predetermined time to react the nitrogen oxides and ozone. Since dinitrogen pentoxide is produced using the plasma generated with air as a source gas by atmospheric discharge, the systemic resistance can be induced in a relatively easy and inexpensive manner. By diluting dinitrogen pentoxide produced at a high concentration, a plant can be exposed to dinitrogen pentoxide at an optimal concentration, and the plant can be prevented from being impaired. Furthermore, by diluting dinitrogen pentoxide with air whose moisture content has been adjusted so that the moisture content after the dilution is less than a predetermined moisture content, dinitrogen pentoxide can be prevented from reacting with water to change into nitric acid.

In the plant disease resistance inducing method and the plant disease resistance inducing device 10 according to the embodiment of the present invention, nitrogen in the source gas can be efficiently dissociated and the production efficiency of the nitrogen oxides with the plasma can be increased by generating the plasma in the NOₓ production portion 14 at 200°C or higher. Also, the production efficiency of ozone with the plasma can be increased by generating the plasma in the ozone production portion 15 at 50°C or lower. The plant disease resistance inducing method and the plant disease resistance inducing device 10 according to the embodiment of the present invention can produce dinitrogen pentoxide at a high concentration by adjusting the moisture content of the source gas to 1×10¹⁵ cm⁻³ (about 40 ppm) or lower in the source gas production portion 12.

Preferably, in the plant disease resistance inducing method and the plant disease resistance inducing device 10 according to the embodiment of the present invention, the plant is exposed to the gas containing dinitrogen pentoxide under a condition optimal for growth of each plant. In particular, since a high moisture content increases the possibility to impair the plant and a low moisture content decreases the effectiveness of inducing the plant resistance, it is preferable to expose the plant to the gas containing dinitrogen pentoxide at an appropriate moisture content for the plant species. For example, it is preferable that *Arabidopsis thaliana* is exposed to the gas containing dinitrogen pentoxide under a growth-optimal condition of 23°C, 50 to 60% moisture content, and 16 daylight hours. The concentration of dinitrogen pentoxide in the gas for the exposure is preferably 0.1 ppm to 1000 ppm, and preferably about 200 ppm or lower, but it is preferable that the concentration is selected as appropriate depending on the plant species. The exposure time is preferably 10 seconds to 1 hour, particularly preferably within 5 minutes, but it is preferable that the time is selected as appropriate depending on the plant species.

The plant disease resistance inducing method and the plant disease resistance inducing device 10 according to the embodiment of the present invention can also be used as a plant calcium ion response inducing method and device respectively, or a plant resistance gene inducing method and device respectively. The produced gas containing dinitrogen pentoxide can also be used as a plant calcium ion response inducing agent or a plant resistance gene inducing agent.

In the plant disease resistance inducing device 10, the ozone production portion 15 may be disposed between the NOₓ production portion 14 and the mixing portion 16. In other words, instead of air, the gas obtained after production of nitrogen oxides by the plasma may be used as the source gas for producing ozone in the ozone production portion 15. In this case, since the gas obtained after production of ozone contains ozone and the nitrogen oxides produced in the NOₓ production portion 14, the gas obtained after production of ozone is put into the tube and preserved for a predetermined time to produce dinitrogen pentoxide. Furthermore, since only one among the flow control portions 13a and 13b is used, the production cost and the operating cost for the device can be reduced.

### Example 1

An experiment was implemented, in which a plant was exposed to dinitrogen pentoxide to confirm induction of Ca²⁺ signals (calcium ion response) as information of injuries similar to insect damages or physical injuries, using the plant disease resistance inducing device 10 illustrated in FIG. 1. In the experiment, *Arabidopsis thaliana* was observed for a live fluorescence of green fluorescence protein (GFP) signals associated with increase in Ca²⁺ signals using a recombinant that expressed a calcium biosensor GFP calmodulin protein (GCaMP) of *Arabidopsis thaliana.*

First, Ca²⁺ signals that had appeared by exposing the whole body of *Arabidopsis thaliana* to dinitrogen pentoxide were observed. In the experiment, the concentration of dinitrogen pentoxide in the gas for the exposure was set to about 200 ppm or lower, and the exposure time was set to 10 seconds. For comparison, Ca²⁺ signals that had appeared by exposing the whole body of *Arabidopsis thaliana* to dry air were also observed under the same condition. FIG. 2 presents states of *Arabidopsis thaliana* before the exposure, and states of the Ca²⁺ signals 60 seconds after the termination of the exposure.

As can be seen from FIG. 2, Ca²⁺ signals were not observed with the dry air exposure, whereas Ca²⁺ signals were observed with the dinitrogen pentoxide exposure. It was also observed that the Ca²⁺ signals were slowly generated over the whole body with the dinitrogen pentoxide exposure.

Next, Ca²⁺ signals that had appeared by exposing one leaf of *Arabidopsis thaliana* to dinitrogen pentoxide were observed. In the experiment, only one leaf of *Arabidopsis thaliana* (leaf surrounded by dotted line in FIG. 3 (a)) was exposed to dinitrogen pentoxide. The concentration of dinitrogen pentoxide in the gas for the exposure was set to about 200 ppm or lower, and the exposure time was set to 10 seconds. The states of the Ca²⁺ signaling up to 6 minutes immediately after the exposure are presented in FIG. 3 (a) to (f).

As presented in FIG. 3 (b), about 20 seconds later, activation of the Ca²⁺ signals (arrow in the figure) was observed in the leaf directly exposed to dinitrogen pentoxide. After that, as presented in FIG. 3 (c) to (f), the Ca²⁺ signals (arrows in each figure) were confirmed to spread to all leaves in the whole body through sieve tubes.

Subsequently, in the experiment presented in FIG. 3, expressions of genes (JAZ5, JAZ7, and OPR3) related to JA synthesis were confirmed by the Ca²⁺ signals to determine whether JA as one of plant hormones had been synthesized. In this experiment, 30 minutes after the dinitrogen pentoxide exposure, a messenger RNA was extracted and purified from leaves that had showed Ca²⁺ signaling (not the leaf surrounded by the dotted line in FIG. 3(a) but the leaves not exposed to dinitrogen pentoxide). Furthermore, a cDNA was synthesized by a reverse transcriptase of the purified messenger RNA. Then, synthetic primer DNAs for three JA-related genes, JAZ5, JAZ7, and OPR3 were used to determine an expression level of each gene as a value relative to an UBQ10 gene expression level by a quantitative real-time PCR (RT-PCR) method.

The sequences of the synthetic primer DNAs used for analyzing the expression levels of the JA-related genes are described below.
JAZ5 gene (AT1G17380: expression regulator gene in JA response gene group)
   Forward sequence: TCATCGTTATCCTCCCAAGC
   Reverse sequence: CACCGTCTGATTTGATATGGG JAZ7 gene (AT2G34600: expression regulator gene in JA response gene group)
   Forward sequence: GATCCTCCAACAATCCCAAA
   Reverse sequence: TGGTAAGGGGAAGTTGCTTG
OPR3 gene (AT2G06050: enzyme gene related to JA biosynthesis: 12-Oxophytodienoate reductase 3)
   Forward sequence: CGTTTTACACTCAAGATCCAGTTG
   Reverse sequence: ATTATCAAACTCAGAGGCGGG
UBQ10 gene (AT4G05320: housekeeping gene used for normalization of gene expression)
   Forward sequence: CACACTCCACTTGGTCTTGCGT
   Reverse sequence: TGGTCTTTCCGGTGAGAGTCTTCA

The determined expression levels of each gene are presented in FIG. 4 (a) to (c). For comparison, expression levels of each gene obtained 30 minutes after the exposure of one *Arabidopsis thaliana* leaf to dry air were also determined under the same condition, and presented as control plots in FIG. 4. The experiment was implemented using three independent individuals, and FIG. 4 presents mean values and standard deviations of the three individuals.

As presented in FIG. 4 (a) to (c), it was confirmed that leaves with no exposure expressed the genes JAZ5, JAZ7, and OPR3 related to the synthesis of jasmonic acid (JA) by the exposure to dinitrogen pentoxide. Also, it was confirmed that the expression levels of the genes were significantly increased 3 to 5 times those of leaves expose to dry air.

From the above experimental results, it is considered that the Ca²⁺ signals propagate to the whole body through the sieve tubes by the dinitrogen pentoxide exposure, and jasmonic acid (JA) as one of plant hormones is synthesized even in distant healthy organs. This may lead to induction of the induced systemic resistance (ISR) defense response.

An experiment in which the plant was exposed to ozone instead of dinitrogen pentoxide was also implemented, but leaves were confirmed to curl up immediately after the ozone exposure and wither the next day. In contrast, leaves exposed to dinitrogen pentoxide were confirmed not to curl up but to grow without withering.

### Example 2

A plant was exposed to dinitrogen pentoxide using the plant disease resistance inducing device 10 illustrated in FIG. 1, and a gene expression was analyzed by RNA sequencing (RNA-seq) and quantitative real-time PCR (qRT-PCR). In the RNA-seq analysis, *Arabidopsis thaliana* at 5 week of seeding was exposed to dinitrogen pentoxide once a day for 3 days. In each exposure, *Arabidopsis thaliana* was exposed to dinitrogen pentoxide from a distance of 5 cm for 20 seconds. A concentration of dinitrogen pentoxide in the gas for the exposure was set to about 200 ppm or lower. Twenty-four hours after the last exposure, leaves that had fully developed were collected, from which the total RNA was extracted, followed by RNA-seq analysis. For comparison, as a control plot, a leaf exposed to dry air under the same condition was also subjected to the RNA-seq analysis.

Genes with expression levels not less than twice as high as that of the control plot, induced by the dinitrogen pentoxide exposure, and genes with reduced expression levels not more than half as high as that of the control plot were extracted. As a result, 1304 genes with twice or more of induced expression levels, and 1209 genes with expression levels reduced to half or less were found. A result of a gene ontology analysis suggested that jasmonic acid signals were activated by the dinitrogen pentoxide exposure.

Based on the result of the RNA-seq, six genes: PDF1.2, ORA59, PAD3, CYP71A12, NIT2, and WRKY26, were selected as candidate marker genes for the dinitrogen pentoxide exposure. Herein, PDF 1.2 is an antimicrobial peptide gene and is often used as a marker gene for jasmonic acid and ethylene. ORA59 is a gene related to regulation of PDF 1.2 expression. PAD3 is a gene related to biosynthesis of an antimicrobial substance camalexin, which is synthesized and induced by jasmonic acid. CYP71A12 is a gene related to a tryptophan metabolism at an upstream of PAD3. NIT2 is a gene related to biosynthesis of auxin that is another plant hormone derived from tryptophan. WRKY26 is a gene which, as is known, functionally overlaps with a transcription factor WRKY33 that regulates jasmonic acid signals.

The results of the RNA-seq analysis for each of the selected genes are presented in Table 1. As presented in Table 1, it was confirmed that each of the selected genes had an expression level about 3 times to 1000 times ("At-N205-20s" in the table) as high as that of the control plot (dry air) by the dinitrogen pentoxide exposure.

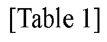

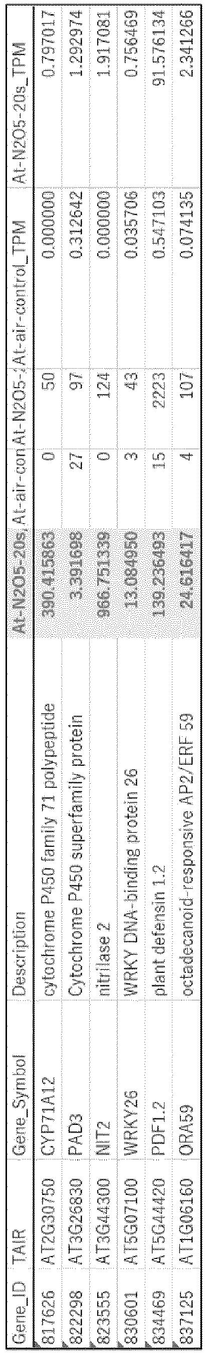

Subsequently, the expression of each of the selected genes was analyzed by qRT-PCR. In the qRT-PCR, *Arabidopsis thaliana* at 5 week of seeding was exposed to dinitrogen pentoxide from a distance of 5 cm for 20 seconds. The dinitrogen pentoxide exposure was conducted only once. The whole above-ground part was sampled 1, 3, 6, 12, 24, and 48 hours after the exposure, from which the total RNA was extracted and then subjected to qRT-PCR. Using ACT2 gene as a control for endogenous genes, an expression level of each gene was determined as a value relative to the ACT2 gene expression level (Relative expression to ACT2 gene).

The primer sequences of genes used for qRT-PCR are described below.
PDF1.2 gene (AT5G44420)
   PDF1.2-F2: ATGCATGCAAGAATCAGTGC
   PDF1.2-R1: AATACACACGATTTAGCACC
ORA59 gene (AT1G06160)
   ORA59 F: ACAACACTTGTGGTTCTTGAGG
   ORA59 R: TACACGGCCATCACATCTCTTC
PAD3 gene (AT3G26830)
   PAD3 F: AGCCTTCTTTCAAGTTCTTCACC
   PAD3 R: CAGATATTTAGAAGCTGACTCCAAC
CYP71A12 gene (AT2G30750)
   CYP71A12 q F: TAGACAGGATCAACGGTTTCAA
   CYP71A12 q R: TGGAATCCAATACTCTTCTCGC
NIT2 gene (AT3G44300)
   NIT2 F2: CGTCAGTTCATGTCACTCCTATG
   NIT2 R2: TGGTTGTAGAATGTAGGGTAACAC
WRKY26 gene (AT5G07100)
   3015 WRKY26: CTGATGACGGCTACAATTGGC
   3016 WRKY26: TGCATTTGAAGTAACTCCTCGG
ACT2 gene (AT3G18780) actin
   ACT2 F: GGTAACATTGTGCTCAGTGGTGG
   ACT2 R: GGTGCAACGACCTTAATCTTCAT

Time-dependent changes in the determined expression levels of each gene are presented in FIG. 5 (a) to (f). For comparison, time-dependent changes in the expression levels of each gene in *Arabidopsis thaliana* exposed to dry air under the same condition were also determined, and the results are presented in FIG. 5. For the experiment, three independent individuals were used, and the mean values and standard deviations of the three individuals are presented in FIG. 5. The same experiment was conducted twice independently, and the same results were obtained.

As presented in FIG. 5 (a) to (c), it was confirmed that the expressions of WRKY26, CYP71A12, and PAD3 were transiently induced in 1 to 3 hours after the dinitrogen pentoxide exposure. As presented in FIG. 5 (d), it was confirmed that the NIT2 expression was transiently induced with a peak at 12 hours after the exposure. As presented in FIG. 5 (e), it was confirmed that the ODR59 expression was induced from 1 hour after the exposure, and a high expression level was maintained until 48 hours had elapsed. As presented in FIG. 5 (f), it was confirmed that the PDF1.2 expression was induced from 6 hours after the exposure, and a high expression level was maintained at least until at least 48 hours had elapsed.

These results suggested that the dinitrogen pentoxide exposure activated the transcription factor related to j asmonic acid signals, the antimicrobial peptide genes, the antimicrobial substance camalexin biosynthesis, and the like. This suggests that the induced systemic resistance (ISR) defense response is induced to enhance the disease resistance.

### Example 3

A plant was exposed to dinitrogen pentoxide using the plant disease resistance inducing device 10 illustrated in FIG. 1, and subjected to an experiment for examining an effect of suppressing viral growth. In the experiment, *Arabidopsis thaliana* at 5 week of seeding was exposed to dinitrogen pentoxide once a day for 3 days. In each exposure, *Arabidopsis thaliana* was exposed to dinitrogen pentoxide from a distance of 5 cm for 20 seconds. A concentration of dinitrogen pentoxide in the gas for the exposure was set to about 200 ppm or lower. Twenty-four hours after the last exposure, leaves that had fully developed were inoculated with a cucumber mosaic virus (CMV) macula lutea strain by a friction inoculation method. Two days after the CMV inoculation, viral loads in the inoculated leaves were quantitatively measured by Enzyme-Linked Immuno Sorbent Assay (ELISA) method using an anti-CMV coat protein antibody.

The quantitatively measured virus loads (Accumulation of virus coat protein) are presented in FIG. 6. For comparison, virus loads of *Arabidopsis thaliana* exposed to dry air and inoculated with CMV under the same condition, and *Arabidopsis thaliana* exposed to dry air and not inoculated with CMV (Mock) under the same condition were also quantitatively measured, and the results are presented in FIG. 6. For the experiment, six independent individuals were used, and the mean values and standard deviations of the six individuals are presented in FIG. 6. The same experiment was conducted three times independently, and the same results were obtained.

As presented in FIG. 6, it was confirmed that the CMV growth was significantly suppressed in the case of the dinitrogen pentoxide exposure compared to the case of the dry air exposure. This suggests that the dinitrogen pentoxide exposure induces the plant immune system to enhance the disease resistance.

### Reference Signs List

10: Plant disease resistance inducing device
11: Source gas introduction portion
12: Source gas production portion
13a, 13b: Flow control portion
14: NOₓ production portion
15: Ozone production portion
16: Mixing portion
17: Dilution portion
   17a: Dryer
   17b: Flow controller

## Claims

1. A plant disease resistance inducing method, wherein a plant is exposed to a gas containing dinitrogen pentoxide to induce a disease resistance.

2. The plant disease resistance inducing method according to claim 1, wherein dinitrogen pentoxide is produced using a plasma generated with air as a source gas, and a plant is exposed to a gas containing dinitrogen pentoxide.

3. The plant disease resistance inducing method according to claim 1 or 2, comprising:
a NOₓ production step of producing nitrogen oxides by generating a plasma using, as a source gas, a gas containing nitrogen and oxygen;
an ozone production step of producing ozone by generating a plasma using, as a source gas, a gas containing nitrogen and oxygen, or a gas obtained after the plasma generation in the NOₓ production step;
a mixing step of producing dinitrogen pentoxide by preserving the nitrogen oxides produced in the NOₓ production step and ozone produced in the ozone production step in a same space for a predetermined time; and
a dilution step of diluting dinitrogen pentoxide by adding a gas containing nitrogen and oxygen, conditioned such that its moisture content after dilution is lower than a predetermined moisture content, to dinitrogen pentoxide produced in the mixing step, wherein
a plant is exposed to a gas containing dinitrogen pentoxide produced in the dilution step.

4. A plant disease resistance inducing device, which can expose a plant to a gas containing dinitrogen pentoxide to induce a disease resistance.

5. The plant disease resistance inducing device according to claim 4, comprising
a dinitrogen pentoxide production portion that can produce dinitrogen pentoxide using a plasma generated with air as a source gas, wherein
the plant can be exposed to the gas containing dinitrogen pentoxide produced in the dinitrogen pentoxide production portion.

6. The plant disease resistance inducing device according to claim 4 or 5, comprising:
a NOₓ production portion that can produce nitrogen oxides by generating a plasma using, as a source gas, a gas containing nitrogen and oxygen;
an ozone production portion that can produce ozone by generating a plasma using, as a source gas, a gas containing nitrogen and oxygen, or a gas obtained after the plasma generation in the NOₓ production portion;
a mixing portion that can produce dinitrogen pentoxide by preserving the nitrogen oxides produced in the NOₓ production portion and ozone produced in the ozone production portion in a same space for a predetermined time; and
a dilution portion that dilutes dinitrogen pentoxide by adding a gas containing nitrogen and oxygen, conditioned such that its moisture content after dilution is lower than a predetermined moisture content, to dinitrogen pentoxide produced in the mixing portion, wherein
a plant can be exposed to a gas containing dinitrogen pentoxide produced in the dilution portion.

7. A plant calcium ion response inducing method, wherein a plant is exposed to a gas containing dinitrogen pentoxide to induce a calcium ion response in cells of the plant.

8. A plant calcium ion response inducing device, which can expose a plant to a gas containing dinitrogen pentoxide to induce a calcium ion response in cells of the plant.

9. A plant resistance gene inducing method, wherein a plant is exposed to a gas containing dinitrogen pentoxide to induce a resistance gene expression of the plant.

10. A plant resistance gene inducing device, which can expose a plant to a gas containing dinitrogen pentoxide to induce a resistance gene expression of the plant.

11. A plant disease resistance inducing agent, comprising a gas containing dinitrogen pentoxide.

12. A plant calcium ion response inducing agent, comprising a gas containing dinitrogen pentoxide.

13. A plant resistance gene inducing agent, comprising a gas containing dinitrogen pentoxide.
